**Europäisches Patentamt**

(19) **European Patent Office**

**Office européen des brevets**

(11) Veröffentlichungsnummer: **0 143 416**
**B1**

(12) # EUROPÄISCHE PATENTSCHRIFT

(45) Veröffentlichungstag der Patentschrift:
08.04.87

(51) Int. Cl.⁴: **C 07 C 139/14,** C 07 C 143/02,
C 01 B 17/90

(21) Anmeldenummer: 84113952.0

(22) Anmeldetag: 17.11.84

(54) Verfahren zur Isolierung von Alkalisulfat-armen Paraffinsulfonaten und Schwefelsäure aus Paraffin-Sulfoxidation-Reaktionsgemischen.

(30) Priorität: 28.11.83 DE 3342984

(43) Veröffentlichungstag der Anmeldung:
05.06.85 Patentblatt 85/23

(45) Bekanntmachung des Hinweises auf die Patenterteilung:
08.04.87 Patentblatt 87/15

(84) Benannte Vertragsstaaten:
BE DE FR GB IT NL

(56) Entgegenhaltungen:
EP-A-0 037 883
DE-A-2 139 477

(73) Patentinhaber: HOECHST AKTIENGESELLSCHAFT,
Postfach 80 03 20, D-6230 Frankfurt am Main 80
(DE)

(72) Erfinder: Pistorius, Rudolf, Dr., Neumühlstrasse 15,
D-6274 Hünstetten (DE)

EP 0 143 416 B1

## Beschreibung

Die durch Sulfoxidation von n-Paraffinen z.B. nach dem Verfahren des deutschen Patents 910 165 erhältlichen wässrigen Lösungen von Paraffinsulfonsäuren enthalten außerdem noch Schwefeldioxid, Schwefelsäure und hydrotrop gelöste Paraffine. Um aus solchen Reaktionsgemischen brauchbare Paraffinsulfonsäuren bzw. Paraffinsulfonate von guter Qualität, d.h. möglichst schwefelsäure- bzw. salzarme, helle und weitestgehend geruchsfreie Produkte zu isolieren, müssen Schwefeldioxid, Schwefelsäure und Paraffine möglichst quantitativ und schonend abgetrennt werden. Die Paraffinsulfoxidationsprodukte beginnen sich bereits bei Temperaturen oberhalb 50° C zu zersetzen, was sich äußerlich an der Verfärbung des sauren Reaktionsgemisches von wasserhell über gelblich, braun bis schließlich tiefschwarz zeigt. Wenn auch die Menge der durch die Temperatureinwirkung zersetzten Paraffinsulfonsäure noch relativ gering ist, solange die sauren Reaktionsgemische nicht über längere Zeit Temperaturen über 100° C ausgesetzt sind, erfordert jedoch bereits ein kleiner Anteil an zersetzten Produkten wegen ihrer Farbintensität einen beträchtlichen Bleichaufwand, wenn man zu einwandfrei hellen Produkten kommen will.

Es wurde festgestellt, daß demgegenüber alkalisch reagierende Salze der Paraffinsulfonsäuren relativ stabil sind. Temperaturen unter 200° C führen auch bei längerer Erhitzungsdauer nur zu ganz unwesentlichen Verfärbungen und auch höhere Temperaturen bis zu etwa 260° C ergeben Verfärbungen, die sich noch leicht mit geringen Bleichmittelmengen wieder beseitigen lassen.

Es muß daher bereits beim ersten Schritt der Aufarbeitung der Paraffin-Sulfoxidations-Reaktionsgemische, d.h. bei der Entgasung zur Entfernung des Schwefeldioxids, darauf geachtet werden, daß möglichst keine Verfärbung auftritt. Wird die Entgasung im schwachen Vakuum ausgeführt, so bedarf es nur eines sehr kurzzeitigen Erwärmens auf ca. 85° C, um eine nahezu vollständige Eliminierung des Schwefeldioxids zu erreichen. Ebenso möglich ist ein Ausblasen mit Inertgas oder auch mit reinem Sauerstoff in einer mit einet geeigneten Packung gefüllten Säule bei einer Temperatur von ca. 40- 70° C.

Durch unmittelbar anschließendes Wiederabkühlen des Reaktionsgemisches auf Raumtemperatur kann bei diesem Prozeßschritt eine merkliche Zersetzung, d.h. eine eintretende Farbvertiefung des Reaktionsgemisches verhindert werden.

Im Hinblick auf die Qualität des Paraffinsulfonats wäre es günstig, das Reaktionsgemisch nach der Entgasung sofort zu neutralisieren. Eine derartige Verfahrensweise ist jedoch wegen des zur Neutralisierung der Schwefelsäure erforderlichen hohen Verbrauchs an Alkali sowie wegen der beachtlichen Verluste an Paraffinsulfonat, die beim Abfiltrieren des Alkalisulfats auftreten, unwirtschaftlich und technisch aufwendig.

Nach der Entfernung des Schwefeldioxids aus dem Reaktionsgemisch muß deshalb versucht werden, vor der Neutralisation die Schwefelsäure möglichst vollständig und unter Schonung der Paraffinsulfonsäure aus dem Gemisch abzutrennen. Bei den bekannten Verfahren, die ein solches Ziel anstreben, geht man im allgemeinen so vor, daß man das entgaste Sulfoxidationsgemisch mit einem geeigneten organischen Lösungsmittel behandelt, um eine Entmischung in eine organische Phase, welche die Paraffinsulfonsäuren enthält und in eine wäßtige Phase, welche die Schwefelsäure soweit wie möglich in Form einer im allgemeinen 10 bis 25%igen wäßrigen Lösung enthält, herbeizuführen. Die beiden Phasen werden dann getrennt und die organische Phase zut Isolierung det Paraffinsulfonsäuren bzw. ihrer Salze weiter aufgearbeitet. So ist es aus der am 29.1.1953 bekanntgemachten deutschen Patentanmeldung F 3718, 120 bereits bekannt, wasserunlösliche oder nur beschränkt mit Wasser mischbare organische Lösungsmittel, wie z.B. Benzol, Chlorbenzol, Cyclohexan, Tetrachlorkohlenstoff, Chloroform, Methylenchlorid und dergleichen zur Abtrennung von Schwefelsäure dem Sulfoxidationsgemisch zuzusetzen. Gemäß DE-OS 27 30 245 kommen für den gleichen Zweck auch Ether wie z.B. Diethylether oder Di-n-butylether und gemäß DE-OS 27 45 691 auch Ketone oder Ester sowie gemäß DE-OS 21 39 477 auch Alkohole mit mindestens 5 Kohlenstoffatomen zur Anwendung.

Keines dieser bekannten Verfahten zur Abtrennung von Schwefelsäure bei niederen Tempetaturen hat sich bislang groß-technisch durchsetzen können, weil entweder der Aufwand bei der destillativen Aufarbeitung det Produktlösung zu groß und/oder der Abscheidungsgrad der Schwefelsäure aus dem Reaktionsgemisch nicht ausreichte, um schließlich zu salzarmen Produkten zu gelangen, die weniger als 2 Gew.-% Restsalz (bezogen auf 100 % Paraffin-Sulfonat) enthalten.

So kann man z.B. mit Alkoholen mit 4 bis 6 C-Atomen bei einstufiger Extraktion die Schwefelsäure nur so unvollständig abtrennen, daß im neutralisierten Endprodukt der Salzgehalt immer noch wesentlich über 2 Gew.-% (bezogen auf Paraffin-Sulfonat) liegt, auch wenn die zugesetzte Alkoholmenge auf 30 Gew.-% (bezogen auf das entgaste Sulfoxidations-Reaktionsgemisch) gesteigert wird. Größere oder kleinere Alkoholmengen führen dagegen zu einer noch weniger vollständigen Abscheidung der Schwefelsäure.

Gibt man jedoch z.B. nach der Abscheidung mit Hexanol nochmals Wasser hinzu, un weitere Schwefelsäure aus dem Reaktionsgemisch auszuwaschen (2-stufige Extraktion), damit det Restsalzgehalt im Paraffinsulfonat schließlich 2 Gew.-% (bezogen wiederum auf die waschaktive Substanz) nicht übersteigt, so stellt man fest, daß hierzu nicht unbeträchtliche Wassermengen erforderlich sind, die sich zudem nur zu einem kleineren Teil wieder abscheiden, wodurch der destillative Aufwand sehr stark ansteigt.

Einerseits nimmt der Grad der Schwefelsäureabscheidung mit steigender C-Zahl der eingesetzten Alkohole zu, andererseits wächst aber dadurch der Aufarbeitungsaufwand mit dem Ansteigen der Siedepunkte der verwendeten Alkohole.

Gegenstand der Erfindung ist ein Verfahren zur Isolierung von Alkalisulfat-armen Paraffinsulfonaten und Schwefelsäure aus Paraffin-Sulfoxidation-Reaktionsgemischen mit Hilfe von Alkoholen, das darin besteht, daß man das von Schwefeldioxid befreite Reaktionsgemisch mit einem $C_4$-$C_8$-Alkohol versetzt, die sich abscheidende untere Phase von verdünnter Schwefelsäure abtrennt, die verbleitende obere Produktphase (1) mit einer solchen Menge an Alkalihydroxid versetzt, bis sich zwei Phasen bilden, die so erhaltene obere Produktphase (2) durch Zugabe einer Teilmenge der Produktphase (1) auf einen pH-Wert von 9-12 einstellt und durch Eindampfen aufkonzentriert.

Ausgangspunkt ist das bei der Sulfoxidation von n-Paraffinen, insbesondete von $C_{13}$-$C_{18}$-Paraffinen erhaltene, durch Entgasung vom Schwefeldioxid befreite Reaktionsgemisch, das bei Temperaturen von 15 bis 80° C, insbesondere bei 25 bis 35° C mit 15 bis 30, insbesondere 17 bis 25 Gew.-% eines $C_4$-$C_8$-Alkohols verrührt wird. Bevorzugt ist hierbei Iso-butanol.

Nach 5 bis 35 Minuten, im allgemeinen bereits nach ca. 15 Minuten, trennt sich ein solches Gemisch in 2 Phasen auf, von denen die untere, die ca. 15-20 %ige wäßrige Schwefelsäure mit ca. 0,1 bis 3 Gew.-% des Alkohols enthält, abgetrennt wird.

Der größere Teil (ca. 60 bis 75 %) det oberen Paraffinsulfonsäure/Alkohol-haltigen Phase wird mit soviel 45-55%iger, im allgemeinen 50%iger Kali- oder Natronlauge versetzt, daß sich bei 80-90° eine untere Phase abscheidet, die einen Teil der überschüssigen Base und den größten Teil det Restschwefelsäure in Form von Alkalisulfat enthält. Die so vom Restsalz weitestgehend befreite, jedoch noch überschüssiges Alkali enthaltende Paraffinsulfonat-Lösung wird durch Zugabe der restlichen 25-40% der Paraffinsulfonsäure/Alkohol-Phase auf einen pH-Wert von ca. 9 tis 12, im allgemeinen 11 (gemessen mit einer Glaselektrode) eingestellt. Die so erhaltene Lösung wird anschließend in einem Dünnschichtvetdampfer im Vakuum im Gegenstrom zur Schmelze eingedampft.

Bei der so geschilderten Verfahrensweise würde eine wäßrige Alkalisulfat/Alkalilösung anfallen, was zu einem unerwünscht hohen Alkalivertrauch führt. Führt man die Behandlung mit überschüssigem Alkali jedoch 3-stufig durch, wie im folgenden beschrieben, so wird das Alkali vollständig für die Neutralisation ausgenutzt und es fällt lediglich eine wäßrige Natriumsulfatlösung an, aus der sich erforderlichenfalls das Sulfat leicht durch Zusatz von z.B. $CaCl_2$ als Gips ausfällen und damit abtrennen läßt.

Ausgangsprodukt ist hier ebenfalls das entgaste Paraffin-Sulfoxidation-Reaktionsgemisch bzw. die nach Zugabe des Alkohols anfallende Paraffinsulfonsäure/Alkohol-Phase wie oben geschildert. Diese Lösung wird kontinuierlich in eine Apparatur eingegeben, die aus drei kombinierten Misch-Absetzgefäßen (sogen. Mixer-Settlern) und einem weiteren Mischgefäß bestehen. Die Mixer-Settler sind so dimensioniert, daß sich im jeweiligen Settler-Teil eine Verweilzeit von ca. 10 bis 20 Minuten einstellt. Alle 3 Mixer-Settler werden tei 80-90° C betrieben.

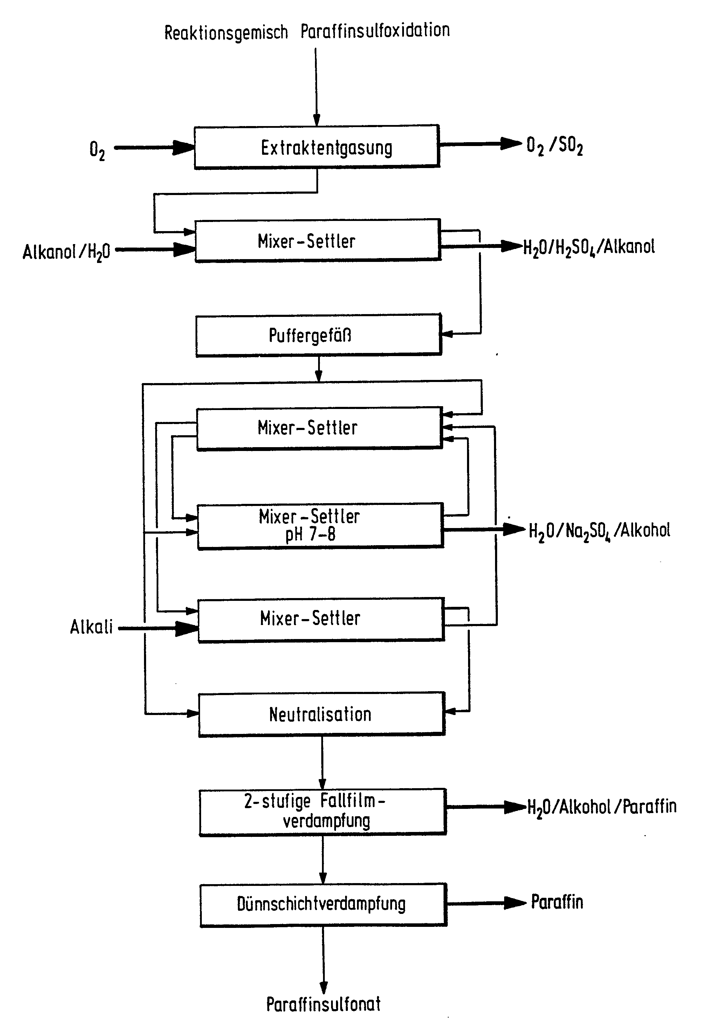

In den 1. Mixer werden die Paraffinsulfonsäure/Alkohol-Lösung, die Oberphase des 2. Settlers und die Unterphase des 3. Settlers eindosiert, in den 2. Mixer die Unterphase des 1. Settlers und soviel von der Paraffinsulfonsäure/Alkohol-Lösung, daß der pH-Wert im 2. Mixer-Settler immer zwischen 7 und 8 liegt. Der 3. Mixer nimmt die gesamte benötigte Alkalilauge auf und die Produktphase des 1. Settlers. Ausgeschleust werden aus dem 2. Settler die Unterphase, die aus einer wäßrigen Alkalisulfatlösung mit einer Spur Alkohol (unter 1 Gew.-%) besteht und die obere Phase aus dem 3. Settler, die im Mischgefäß mit weiterer Paraffinsulfonsäure/Alkohol-Lösung auf den pH-Wert von ca. 11 eingestellt wird. Diese Lösung wird dann bis zu dem gewünschten Konzentrationsgrad eingedampft. Das Fließschema dieser Variante des beanspruchten Verfahrens ist in der Zeichnung dargestellt. Beiden Varianten ist als wesentliches Merkmal gemeinsam, daß die weitgehende Abtrennung der Schwefelsäure bzw. des Sulfats durch die erneute Bildung von zwei Phasen infolge der Zugabe von überschüssigem Alkalihydroxid erreicht wird.

Ein wesentlicher Vorteil des erfindungsgemäßen Verfahrens ist darin zu sehen, daß es gelingt, helle und geruchsarme Produkte auf sehr wirtschaftliche Weise zu erhalten, was vor allem durch die Abscheidung des überwiegenden Teils der Schwefelsäure unter außerordentlich milden Bedingungen bewirkt wird. Durch die oben geschilderte Verfahrensweise bei der Neutralisation der Paraffinsulfonsäure zu Paraffinsulfonaten gelingt es durch die Ausschleusung von weiterem wäßrigem Natriumsulfat gleichzeitig extrem salzarme Produkte zu erhalten.

**Beispiel 1**

Verwendet wird ein Sulfoxidations-Reaktionsgemisch der Zusammensetzung
41.0 % $H_2O$
7.13 % $H_2SO_4$

20.38 % $RSO_3H$

31.49 % Paraffin

Bei dem hier und in den folgenden Beispielen benutzten Paraffin handelt es sich um ein Gemisch von $C_{13}$-$C_{17}$-Paraffinen. Dementsprechend bedeutet R ein Gemisch von $C_{13}$ bis $C_{17}$-Alkyl.

1000 g des Reaktionsgemisches werden mit 200 g wassergesättigtem Hexanol bei Raumtemperatur versetzt, wobei sich als untere Phase 369,2 g verdünnte wäßrige Schwefelsäure abscheidet.

Die obere Phase (Paraffinsulfonsäure/Hexanol-Lösung) enthält dann 0.73 % Schwefelsäure und 24,86 % Paraffinsulfonsäure.

Von dieser Phase werden 400 g 5 Minuten lang mit 64 g 50%iger Natronlauge bei 80°C gerührt.

Nach einer Abscheidezeit von 15 Minuten trennen sich 52 g als wäßrige Unterphase, die 4,5 % $Na_2SO_4$ und 18 % NaOH enthält. Die obere Produktphase wird mit 119 g der Paraffinsulfonsäure/Hexanol-Lösung auf den pH-Wert von 11 eingestellt und im Dünnschichtverdampfer bei 30 Torr und 230°C Heizöltemperatur zur Schmelze eindampft, die die folgende Zusammensetzung aufweist:

97.6 % $RSO_3Na$

0.8 % Paraffin

1.6 % $Na_2SO_4$

**Beispiel 2**

1000 g des gleichen Sulfoxidations-Reaktionsgemisches wie im Beispiel 1 werden mit 200 g Isobutanol und 34 g Wasser 2 Minuten bei 28°C gerührt und 3 h stehen gelassen.

Die obere Phase enthält dann 0,87 % Schwefelsäure und 24,01 % Paraffinsulfonsäure. 400 g hiervon werden 3 Minuten mit 68 g 50%iger Natronlauge gerührt und 15 Minuten bei 85-88°C stehen gelassen. Es scheiden sich als wäßrige Phase 69 g ab, die 17,6 % Natronlauge und 6,1 % Natriumsulfat enthält. Mit 114 g Paraffinsulfonsäure-Isobutanol-Lösung wird die obere Phase anschließend auf pH = 11 gestellt und danach bei 30 Torr im Dünnschichtverdampfer eingedampft (Heizöltemperatur 230°C). Die erhaltene Schmelze hat folgende Zusammensetzung:

97,4 % $RSO_3Na$

0.8 % Paraffin

1.8 % $Na_2SO_4$

**Beispiel 3**

10,28 kg Reaktionsmischung mit der Zusammensetzung

4240 g $H_2O$

2160 g $RSO_3H$

740 g $H_2SO_4$

3140 g Paraffin

werden mit 2060 g Isobutanol, 500 g $H_2O$ und 50 g Paraffin versetzt. Nach einer Stunde wurden als Unterphase 4020 g abgetrennt, die 3240 g Wasser, 660 g Schwefelsäure und 120 g Isobutanol enthält. Die Schwefelsäure-arme Oberphase (8870 g) besteht aus 1500 g $H_2O$, 2160 g Paraffinsulfonsäure, 80 g Schwefelsäure, 3190 g Paraffin und 1940 g Isobutanol. Von dieser Phase wurden kontinuierlich in eine Apparatur, bestehend aus 3 Mixer-Settlern, die bei 85-90°C betrieben werden, und einem nachgeschalteten Mischapparat pro Stunde 500 g in den 1. Mixer eindosiert, in den zugleich die Oberphase des 2. Settlers (ca. 234 g/h) und die Unterphase des 3. Settlers fließen (ca. 195 g/h). Gleichzeitig werden in den 3. Mixer 84 g 50%-ige Natronlauge und die Oberphase des 1. Settlers (ca. 800 g/h) eindosiert, während pro Stunde ca. 689 g/h (Zusammensetzung: 123 g $H_2O$, 173 g $RSO_3H$, 1 g $Na_2SO_4$, 238 g Paraffin, 143 g Isobutanol und 11 g NaOH) det Oberphase des 3. Settlers in den Mischapparat fließen, die mit 228 g/h Schwefelsäure-armer Oberphase (Zusammensetzung 39 g $H_2O$, 56 g $RSO_3H$, 2 g $H_2SO_4$, 81 g Paraffin und 50 g Isobutanol) auf den pH-Wert von ca. 11 eingestellt werden. Es resultiert eine Isobutanol-Paraffin-Paraffinsulfonatlösung der Zusammensetzung: 168 g $H_2O$, 233 g $RSO_3Na$, 4 g $Na_2SO_4$, 319 g Paraffin und 192 g Isobutanol (917 g/h), die auf einem Dünnschichtverdampfer bei 30 Torr eingedampft werden (Heizflüssigkeitstemperatur 230°C). Es resultieren 236 g Paraffinsulfonat, die noch 4 g $Na_2SO_4$ und 2 g Paraffin enthalten.

In den 2. Mixer fließt die Oberphase des 1. Settlers (ca. 129 g/h), die mit soviel Schwefelsäure-armer Oterphase (ca. 159 g/h -Zusammensetzung 26 g $H_2O$, 31 g $RSO_3H$, 1 g $H_2SO_4$, 59 g Paraffin und 35 g Isobutanol) vermischt wird, daß sich ein pH-Wert von 7-8 einstellt. Die Unterphase des 2. Settlers besteht aus wäßriger Natriumsulfatlösung (54 g/h) (Zusammensetzung 45 g $H_2O$, 8 g $Na_2SO_4$ und 1 g Isobutanol), die ausgeschleust wird.

**Patentansprüche**

1. Verfahren zur Isolierung von Alkalisulfat-armen Paraffinsulfonaten und Schwefelsäure aus Paraffin-Sulfoxidation-Reaktionsgemischen mit Hilfe von Alkoholen, dadurch gekennzeichnet, daß man das von Schwefeldioxid befreite Reaktionsgemisch mit einem $C_4$-$C_8$-Alkohol versetzt, die sich abscheidende untere Phase von verdünnter Schwefelsäure abtrennt, die verbleibende obere Produktphase (1) mit einer solchen Menge an Alkalihydroxid versetzt, bis sich zwei Phasen bilden, die so erhaltene obere Produktphase (2) durch Zugabe einer Teilmenge der Produktphase (1) auf einen pH-Wert von 9-12 einstellt und durch Eindampfen aufkonzentriert.

2. Verfahren nach Anspruch 1, dadurch gekennzeichnet, daß man die verbleibende obere Produktphase (1) zusammen mit der Unterphase aus einem Absetzgefäß (3) und der Oberphase aus einem Absetzgefäß (2) in ein Absetzgefäß (1) gibt, die im Atsetzgefäß (1) erhaltene Oberphase zusammen mit einer solchen Menge an Alkalihydroxid in das Absetzgefäß (3) gibt, bis sich zwei Phasen bilden; die im Absetzgefäß (1) erhaltene Unterphase gleichzeitig mit einer solchen Menge der Produktphase (1) in ein Absetzgefäß (2) gibt, daß ein pH-Wert von 7 bis 8 aufrechterhalten wird, die im Absetzgefäß (2) erhaltene Oberphase in das Absetzgefäß (1) gibt, die im Absetzgefäß (2) erhaltene Unterphase bestehend im wesentlichen aus einem Wasser/Na$_2$SO$_4$/C$_4$-C$_8$-Alkanol-Gemisch, aus dem Verfahren abtrennt, die im Absetzgefäß (3) erhaltene Unterphase zusammen mit der Produktphase (1) in das Absetzgefäß (1) gibt, die im Absetzgefäß (3) erhaltene Oberphase durch Zugabe einer Teilmenge der Produktphase (1) auf einen pH-Wert von 9-12 einstellt und durch Eindampfen aufkonzentriett.

## Claims

1. A process for isolating paraffinsulfonates and sulfuric acid of low alkali metal sulfate content from paraffinsulfoxidation reaction mixtures with the aid of alcohols, which comprises adding a C$_4$-C$_8$-alcohol to the reaction mixture, which has been freed from sulfur dioxide, removing the lower phase of dilute sulfuric acid which separates out, adding to the remaining upper product phase (1) an amount of alkali metal hydroxide such that two phases form and bringing the upper product phase (2) thus obtained to a pH value of 9-12 by addition of some of the product phase (1) and concentrating it by evaporation.

2. The process as claimed in claim 1, wherein the upper product phase (1) which remains is introduced into a settling vessel (1), together with the lower phase from a settling vessel (3) and the upper phase from a settling vessel (2), the upper phase obtained in the settling vessel (1) is introduced into the settling vessel (3), together with an amount of alkali metal hydroxide such that two phases form, the lower phase obtained in settling vessel (1) is introduced into a settling vessel (2) at the same time as an amount of product phase (1) such that a pH value of 7 to 8 is maintained, the upper phase obtained in the settling vessel (2) is introduced into the settling vessel (1), the lower phase obtained in the settling vessel (2), consisting essentially of a water/Na$_2$SO$_4$/C$_4$-C$_8$-alkanol mixture, is removed from the process, the lower phase obtained in the settling vessel (3) is introduced into the settling vessel (1), together with the product phase (1), and the upper phase obtained in settling vessel (5) is brought to a pH value of 9-12 by addition of part of the product phase (1) and is concentrated by evaporation.

## Revendications

1. Procédé pour isoler, à l'aide d'alcools, des paraffine sulfonates pauvres en sulfate alcalin, et de l'acide sulfurique, à partir de mélanges de réaction de sulfoxydation de paraffine, procédé caractérisé en ce qu'on ajoute, au mélange réactionnel débarrassé du bioxyde de soufre, un alcool en C$_4$ à C$_8$, on enlève la phase inférieure d'acide sulfurique dilué qui se sépare, on ajoute à la phase supérieure restante de produit (1) une quantité d'hydroxyde alcalin telle qu'il se forme deux phases, on ajuste à un pH de 9 à 12, par addition d'une partie de la phase de produit (1), la phase supérieure de produit (2) ainsi obtenu, et on la concentre par évaporation.

2. Procédé selon la revendication 1, caractérisé en ce qu'on introduit la phase supérieure restante de produit (1) avec la phase inférieure provenant d'un récipient de décantation et dépôt (3) et avec la phase supérieure provenant d'un récipient de décantation et dépôt (2) dans un récipient de décantation dépôt (1), on introduit dans le récipient de décantation-dépôt (3) la phase supérieure obtenue dans le récipient de décantation (1), avec une quantité d'hydroxyde alcalin telle qu'il se forme deux phases, on ajoute la phase inférieure obtenue dans le récipient de décantation-dépôt (1) en même temps qu'une quantité de la phase de produit (1) dans un récipient de décantation-dépôt (2) de manière à maintenir un pH de 7 à 8, on ajoute dans le récipient de décantation (1) la phase supérieure obtenue dans le récipient de décantation (2), on enlève du procédé la phase inférieure obtenue dans le récipient de décantation-dépôt (2), phase consistant essentiellement en un mélange d'eau/Na$_2$SO$_4$/alcanol en C$_4$ à C$_8$, on introduit dans le récipient de décantation-dépôt (1) la phase inférieure obtenue dans le récipient de décantation-dépôt (3) avec la phase de produit (1), on ajuste, par addition d'une partie de la phase de produit (1) à un pH de 9 à 12 la phase supérieure obtenue dans le récipient de décantation-dépôt (3) et l'on concentre par évaporation.

Reaktionsgemisch Paraffinsulfoxidation

$O_2$ ⟶ **Extraktentgasung** ⟶ $O_2/SO_2$

Alkanol/$H_2O$ ⟶ **Mixer–Settler** ⟶ $H_2O/H_2SO_4/Alkanol$

**Puffergefäß**

**Mixer–Settler**

**Mixer–Settler pH 7–8** ⟶ $H_2O/Na_2SO_4/Alkohol$

Alkali ⟶ **Mixer–Settler**

**Neutralisation**

**2-stufige Fallfilmverdampfung** ⟶ $H_2O/Alkohol/Paraffin$

**Dünnschichtverdampfung** ⟶ Paraffin

Paraffinsulfonat